# EUROPÄISCHE PATENTSCHRIFT

(11) **EP 0 684 839 B1**
(45) Veröffentlichungstag und Bekanntmachung des Hinweises auf die Patenterteilung: **23.09.1998**
(21) Anmeldenummer: 94906225.1
(22) Anmeldetag: 07.02.1994
(51) Int. Cl.: A61K 47/24, A61K 47/26

(54) **VERFAHREN ZUR HERSTELLUNG KOLLOIDALER WÄSSRIGER LÖSUNGEN SCHWER LÖSLICHER WIRKSTOFFE**
PROCESS FOR PREPARING COLLOIDAL AQUEOUS SOLUTIONS OF HARDLY SOLUBLE ACTIVE SUBSTANCES
PROCEDE DE FABRICATION DE SOLUTIONS AQUEUSES COLLO DALES DE MATIERES ACTIVES DIFFICILEMENT SOLUBLES

(30) Priorität: 18.02.1993 DE 4305003
(43) Veröffentlichungstag der Anmeldung: 06.12.1995
(73) Patentinhaber: KNOLL AKTIENGESELLSCHAFT, D-67061 Ludwigshafen (DE)
(72) Erfinder: ROSENBERG, Joerg, D-67158 Ellerstadt (DE); ROMERDHAL, Cynthia, Wayland, MA 01778 (US); HEBERGER, Juergen, D-67105 Schifferstadt (DE)
(74) Vertreter: Karau, Wolfgang, Dr.
(86) Internationale Anmeldenummer: EP9400332
(87) Internationale Veröffentlichungsnummer: WO9419018

(56) Entgegenhaltungen:
- EP-A- 0 317 120
- EP-A- 0 426 029
- EP-A- 0 456 106
- WO-A-93/01811
- DE-A- 3 010 041
- DATABASE WPI Section Ch, Week 7744, Derwent Publications Ltd., London, GB; Class B04, AN 77-78083Y & JP,A,50 126 821 (SUMITOMO CHEMICAL KK) 6. Oktober 1974

## Beschreibung

Die Verabreichung eines Arzneistoffs durch z.B. Injektion oder Infusion in die Blutbahn scheitert oft an dessen Schwerlöslichkeit in wäßrigen Systemen. Es sind daher in den letzten Jahren verschiedene Verfahren entwickelt worden, um mit Hilfe geeigneter Löslichkeitsvermittler den Arzneistoff in einer wäßrigen Phase zu formulieren.

Die meisten Verfahren nutzen dabei den lösungsfördernden Effekt von Detergentien oder Emulgatoren aus (vgl. Voigt: Lehrbuch der pharmazeutischen Technologie 5. Aufl. Verlag Chemie. S. 334). Obwohl die Herstellung solcher Lösungen sehr einfach ist und eine Vielzahl von Arzneistoffen damit solubilisiert werden können, bereitet die von den Detergentien ausgehende Toxizität oft erhebliche Probleme.

Eine weitere Möglichkeit besteht darin, die Arzneistoffe mit Hilfe von ringförmigen Kohlenhydraten (Cyclodextrinen) zu versetzen, die den Arzneistoff komplexieren (Pharm. Techn. Intern., Februar 1991, S. 15) und die dadurch die Löslichkeit oft entscheidend verbessern. Da der innere Hohlraum der Cyclodextrine aber begrenzt ist, lassen sich viele Moleküle aus sterischen Gründen nicht komplexieren, obwohl die Cyclodextrine toxikologisch günstiger zu bewerten sind als die Detergentien.

Als weitere Klasse von Löslichkeitsvermittlern kommen die Phospholipide infrage, die als körpereigene Moleküle (sie sind Bestandteil einer jeden Zellmembran) eine sehr gute Verträglichkeit auch in Injektionslösungen zeigen. Die ausgezeichnete Verträglichkeit von Phospholipiden führte daher auch zu deren Einsatz bei Formulierungen von Wirkstoffen, die zwar gut wasserlöslich sind, aber wegen nicht tolerabler lokaler Unverträglichkeiten z.B. im Bereich der Venen bei der intravenösen Applikation, Probleme bereiten. Die Verbesserung der lokalen Verträglichkeit durch Wahl von Phospholipidformulierungen ist bekannt (Europ. J. Cancer; Vol. 16, 945-950 (1980). Der Einsatz von Phospholipiden scheitert aber oft daran, daß diese in wäßrigen Systemen nicht molekular, sondern nur unter Bildung kolloidaler Aggregate löslich sind. Diese Kolloidteilchen sind beim einfachen Dispergieren von Phospholipiden in wäßrigen Lösungen jedoch so groß, daß solche phospholipidhaltigen Lösungen für Injektions- und Infusionszubereitungen im allgemeinen nicht einsetzbar sind (Emboliegefahr). Die durch Suspendieren von Phospholipiden in wäßrigen Systemen erhältlichen grobkolloidalen trüben Lösungen müssen daher vor ihrer Verwendung erst mit Hilfe geeigneter Verfahren homogenisiert werden, so daß die Größe der Partikel in den Lösungen auf Werte erniedrigt wird, die einen Einsatz der Phospholipide in Injektions- oder Infusionslösungen erlauben (US 5.008.050).

Ein weiterer Nachteil von Phospholipid-enthaltenden Lösungen besteht darin, daß eine Entkeimung der fertigen Lösungen im Endbehältnis durch Autoklavieren fast immer zum Ausflocken der Phospholipide führt.

Eine Reihe verschiedener Verfahren sind bekannt, um die Partikelgrößen grobkolloidaler Phospholipidlösungen in den Submikrometerbereich zu bringen. Dazu zählen etablierte Labormethoden wie die Ultrabeschallung, aber auch Verfahren im Produktionsmaßstab wie die Hochdruckhomogenisierung. Diese Methoden erfordern jedoch einen relativ hohen Aufwand.

Gegenstand der Erfindung ist ein Verfahren, mit dessen Hilfe schwer lösliche Wirkstoffe zusammen mit Phospholipiden zu einer homogenen Lösung verarbeitet werden können, welches dadurch gekennzeichnet ist, daß man
a) den Wirkstoff mit einem Phospholipid mischt,
b) den Rückstand bei erhöhter Temperatur mit einer stark sauren wäßrigen Lösung eines Kohlenhydrats versetzt und diese Mischung bei erhöhter Temperatur so lange rührt, bis eine homogene kolloidale Lösung entstanden ist und
c) diese Lösung abkühlt und auf einen pH-Wert von 4 bis 5 einstellt und sterilisiert.

Das neue Verfahren eignet sich für basische, in Säuren protonierbare Stickstoffatome aufweisende Wirkstoffe, die im sauren pH-Bereich ionisch vorliegen, wie protonierbare basische Aminogruppen enthaltende Wirkstoffe. Diese Verbindungen besitzen oft eine vergleichsweise gute Wasserlöslichkeit bei stark sauren pH-Werten, kristallisieren aber beim Titrieren auf schwach saure bis neutrale pH-Werte weitgehend in Form der Basen wieder aus.

Insbesondere eignet sich das Verfahren für kationische (protonierbare) Wirkstoffe, die auch in Form ihrer Salze (mit anorganischen oder organischen Gegenionen) eine Löslichkeit in Wasser von weniger als 1 mg/ml besitzen.

Beispiele für solche Wirkstoffe sind die Bisnaphthalimide der US-PS 4,874,683. Diese Bisnaphthalimide sind oft selbst in Form ihrer Salze (z.B. Methansulfonate) in Wasser schwerlöslich (< 1 mg/ml). Bei Erniedrigung der pH-Werts steigt dagegen die Löslichkeit erwartungsgemäß stark an. Lösungen mit diesen niedrigen pH-Werten sind allerdings nicht applizierbar.

Weitere Beispiele sind hochlipophile Wirkstoffe vom Typ des Anipamil-Hydrochlorids, sowie die Wirkstoffe der EP 363 212; beide Wirkstoff-Klassen sind schwerlöslich in Wasser.

Nichtionische Wirkstoffe dagegen lassen sich mit dem erfindungsgemäßen Verfahren nicht umsetzen, da hier die zur Solubilisierung im anfangs stark sauren Medium notwendige Ionisierung wegen des Fehlens einer protonierbaren funktionellen Gruppe nicht möglich ist. Analoges gilt für anionische Wirkstoffe, da diese im stark sauren Milieu zwar protonierbar sind, dann aber nichtionisch vorliegen, wodurch die Wasserlöslichkeit sinkt.

Neben den (bei neutralen pH-Werten) schwerlöslichen kationischen Wirkstoffen ist das Verfahren aber auch für solche Substanzen anwendbar, die bei ausreichender Wasserlöslichkeit Probleme durch lokale Unverträglichkeiten bei der Applikation zeigen (z.B. Venenreizungen bei intravenöser Applikation). Beispiele für derartige Wirkstoffe sind Levemopamid-Hydrochlorid, Dexverapamil-Hydrochlorid sowie wasserlösliche Bisnaphthalimide, die wegen des Fehlens der aromatischen Nitrosubstituenten eine gute Wasserlöslichkeit besitzen.

Geeignet für das erfindungsgemäße Verfahren sind alle natürlichen (d.h. aus Naturrohstoffen isolierten) oder synthetisch hergestellten Phospholipide. Auch Mischungen verschiedener Phospholipide sind möglich. Besonders bevorzugt sind Phospholipidmischungen, die aus Soja oder Eigelb isoliert wurden ("Sojalecithin", "Eilecithin"), insbesondere dann, wenn diese mehr als etwa 80 % Phosphatidylcholin enthalten.

Das Gewichtsverhältnis Phospholipid : Wirkstoff kann 20:1 bis 1:1, vorzugsweise 10 : 1 bis 5 : 1, betragen. Das für den jeweiligen Wirkstoff bestgeeignete Verhältnis muß empirisch ermittelt werden. Der Phospholipidgehalt der fertigen Lösung liegt bei 10 bis 150 mg/ml, bevorzugt bei 20 bis 60 mg/ml.

Eine intensive Vermischung von Wirkstoff und Phospholipid kann im einfachsten Fall durch gemeinsames Lösen in einem organischen Lösungsmittel und anschließendes Entfernen des Lösungsmittels erfolgen. In vielen Fällen reicht ein einfaches Vermischen von Wirkstoff und Phospholipid ohne vorherigen Löseschritt aus.

Als Kohlenhydrate können alle pharmazeutisch gebräuchlichen Mono- und Disaccharide eingesetzt werden. Beispiele dafür sind die in den jeweiligen Pharmakopöen aufgelisteten Stoffe, z.B. Glucose, Fructose etc. Aber auch Zuckeralkohole und andere Polyhydroxyverbindungen sind geeignet. Beispiele dafür sind Sorbit, Xylit, Mannit sowie Glycerin. Bevorzugt sind bei den Kohlenhydraten die Disaccharide, insbesondere Saccharose, Trehalose und Maltose. Bei den Polyhydroxyverbindungen sind Glycerin und Mannit bevorzugt. Die Kohlenhydrate werden den Formulierungen in Mengen zugesetzt, daß die fertigen Lösungen weitgehend blutisoton sind, d.h. sie dienen gleichzeitig zum Isotonisieren der Lösungen auf einen osmotischen Druck, der etwa dem des Bluts entspricht.

Die wäßrigen Lösungen der Kohlenhydrate sollen einen pH-Wert von 1,5 bis 2,5 besitzen. Die Lösung des Gemisches gelingt am besten bei einer Temperatur von 30 bis 60°C.

Die Konstanthaltung der pH-Werte in der fertig titrierten Lösung erfolgt durch Zugabe geeigneter Puffersubstanzen. Es zeigte sich, daß die Stabilität der erfindungsgemäßen Präparationen bei einem pH-Wert von etwa 4 bis 5 am besten ist. Niedrigere pH-Werte können zu Unverträglichkeitsreaktionen bei der Applikation führen (insbesondere bei Injektionen in die Blutbahn), während es bei pH-Werten im neutralen pH-Bereich zu Ausflockungen des Wirkstoffs nach 12 bis 24 Stunden kommt. Somit sind Puffersubstanzen bevorzugt, die eine maximale Pufferkapazität (pKₛ-Wert) im pH-Bereich von 4 bis 5 besitzen. Dazu zählen insbesondere die bei der Arzneiformung gebräuchlichen Mono- und Dicarbonsäuren wie Essigsäure, Bernsteinsäure, aber auch Hydroxycarbonsäuren wie Citronensäure, Weinsäure, Äpfelsäure und Milchsäure, sowie Aminosäuren wie Glycin und Asparaginsäure. Besonders bevorzugt ist Essigsäure. Die Puffersubstanzen werden den erfindungsgemäßen Präparationen vorzugsweise in Konzentrationen von etwa 10 mmol pro Liter zugesetzt.

Zur Verbesserung der Lagerstabilität ist es empfehlenswert, den Lösungen Schwermetall-Chelatoren wie z.B. Ethylendiamintetraessigsäure (EDTA, als Na-Salz) sowie Antioxidantien wie z.B. Tocopherol (bzw. Tocopherol-Derivate) zuzusetzen. Die dabei verwendeten Mengen entsprechen denen, die für pharmazeutisch gebräuchliche Injektionslösungen verwendet werden.

Es war überraschend, daß durch die Anwendung des erfindungsgemäßen Verfahrens phospholipidhaltige Lösungen erhalten werden, in denen die Partikelgrößen der Phospholipid-Aggregate so niedrig liegen, daß im allgemeinen direkt nach der Herstellung eine Sterilfiltration durch 0,2 µm Filter erfolgen kann. Derartige Partikelgrößen in phospholipidhaltigen Lösungen sind ansonsten nur mit aufwendigeren Verfahren erhältlich (Ultrabeschallung, Hochdruckhomogenisierung).

Die abschließende Sterilisation im Endbehältnis kann durch bekannte Verfahren wie z.B. Autoklavieren erfolgen, ohne daß es zum Ausflocken der Phospholipide kommt.

Weiter bleiben die Lösungen über einen langen Zeitraum stabil, ohne daß Ausflockungen auftreten.

Die folgenden Beispiele veranschaulichen die Erfindung. Kriterium für eine gelungene Solubilisierung ist die Möglichkeit zur Sterilfiltration durch ein 0,2 µm Filter.

### Beispiel 1

In der US 4,874,683 sind Wirkstoffe beschrieben, die eine Bisnaphthalimid-Struktur besitzen. Wegen der sehr schlechten Wasserlöslichkeit ist die Herstellung ausreichend konzentrierter wäßriger Wirkstofflösungen sehr erschwert. Unter Zuhilfenahme von Löslichkeitsvermittlern wie Dimethylsulfoxid sind zwar Injektionslösungen herstellbar, aus diesen flockt aber nach der Injektion im Blut der Wirkstoff schnell wieder aus.

Mit einem Derivat dieser Bisnaphthalimide 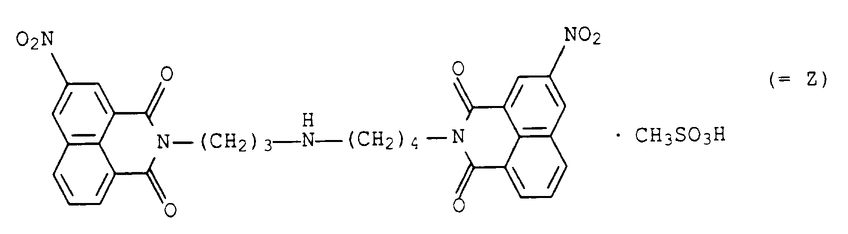 wurde die Löslichkeit wie folgt in wäßrigen Formulierungen untersucht:

### Versuch A (Vergleichsversuch)

40 mg Z wurden in 10 ml Dichlormethan/Methanol (9+1) gelöst. Aus der Lösung wurde am Rotationsverdampfer das Lösungsmittel wieder restlos im Vakuum entfernt. Zum Rückstand gab man 8 ml einer Pufferlösung (bestehend aus o-Phosphorsäure [0,01 M] und Saccharose [0,265 M] und EDTA-Dinatriumsalz [0,1 mg/ml] pH ca. 1,9) und drehte den Kolben 1 h am Rotationsverdampfer (ohne Vakuum) bei 50°C Wassertemperatur. Ein erheblicher Teil des Wirkstoffs konnte so in Lösung gebracht werden. Nach dem Abkühlen auf Raumtemperatur titrierte man mit 2 molarer Natronlauge auf einen pH-Wert von 6,8 und füllte dann auf 10 ml Gesamtvolumen mit der oben genannten Pufferlösung (pH 6,8; titriert mit NaOH) auf. Dabei fiel der Wirkstoff zum größten Teil wieder aus. Der klare Überstand wurde durch ein 0,2 Mikrometer Spritzenfilter filtriert und in dieser Lösung spektralfotometrisch die Wirkstoffkonzentration bestimmt. Der Wirkstoffgehalt dieser Lösung lag unter 0,1 mg/ml.

### Versuch B (Vergleichsversuch)

Der Versuch erfolgte analog Versuch A, aber es wurden zusätzlich zum Wirkstoff 1,0 g Phospholipid (Eierlecithin E 100, Fa. Lipoid KG, Ludwigshafen) eingewogen.

Der Wirkstoffgehalt der auf pH 6,8 titrierten und sterilfiltrierten Lösung betrug 4,0 mg/ml. Die Lösung flockte nach etwa 12 bis 24 h sowohl bei Raumtemperatur als auch im Kühlschrank langsam aus.

### Versuch C

Der Versuch erfolgte analog Versuch B, aber nach Zugabe der Pufferlösung (pH 1,9) und dem Rühren bei 50°C (1 h) wurden 100 Mikroliter einer verdünnten wäßrigen Lösung von Eisessig (120,1 g/l) zugesetzt und diese Lösung dann mit Natronlauge (2 molar) auf einen pH-Wert von 4,6 titriert.

Der Wirkstoffgehalt dieser Lösung betrug nach Sterilfiltration 4,0 mg/ml. Die Lösungen waren ausnahmslos sowohl bei Raumtemperatur als auch im Kühlschrank ohne Ausflockung über Wochen stabil.

### Beispiel 2

Das Beispiel wurde analog Beispiel 1, Versuch C durchgeführt, aber mit 5,5 mg/ml Levemopamil-Hydrochlorid und 30,0 mg Eierlecithin E 100. Der pH-Wert wurde auf pH 4,8 eingestellt.

Die fertige Lösung wurde über eine stufenweise erfolgende Filtration (zuerst 0,45 µm, dann 0,2 µm) entkeimt.

### Vergleichsbeispiel

Mit dem schwerlöslichen, nichtionischen Wirkstoff Esuprone (EP 111.746, Beispiel 30), dessen Wasserlöslichkeit bei lediglich 15 µg/ml liegt, wurde ein Versuch analog Beispiel 1, Versuch C durchgeführt. Es wurde eine grobkolloide Lösung erhalten, die nicht filtrierbar war, weder durch ein 0,2 µm Filter noch durch ein 0,45 µm Filter.

### Beispiel 3

Mit dem schlecht wasserlöslichen, hydrophoben Peptid Dolastatin-15 erfolgte ein Versuch analog dem Vergleichsbeispiel. Der Wirkstoffgehalt betrug 10 mg/ml, der Phospholipidgehalt 100 mg/ml. Die Lösung konnte sterilfiltriert werden.

### Beispiel 4

Das Beispiel 3 wurde wiederholt, jedoch wurde Wirkstoff die Verbindung 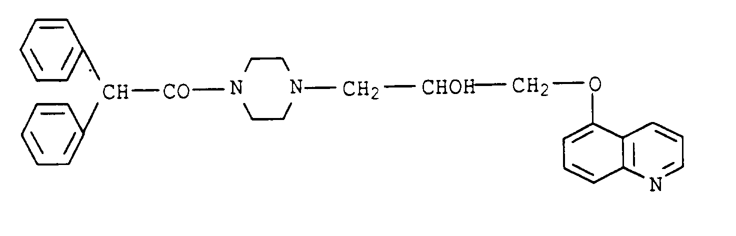 in Form des Mono-fumarats (EP 363.212) eingesetzt. Man erhielt eine sterilfiltrierbare Lösung.

### Beispiel 5

Das Beispiel 3 wurde wiederholt, jedoch wurde als Wirkstoff Dexverapamil-Hydrochlorid (= R-Verapamil x HCl) in einer Menge von 20 mg/ml eingesetzt. Man erhielt eine sterilfiltrierbare Lösung.

## Patentansprüche

1. Verfahren, mit dessen Hilfe basische, in Säuren protonierbare Stickstoffatome aufweisende Wirkstoffe zusammen mit Phospholipiden zu einer homogenen Lösung verarbeitet werden können, welches dadurch gekennzeichnet ist, daß man
a) den Wirkstoff mit einem Phospholipid mischt,
b) den Rückstand bei erhöhter Temperatur mit einer sauren wäßrigen Lösung eines Kohlenhydrats versetzt und diese Mischung bei erhöhter Temperatur so lange rührt, bis eine homogene kolloidale Lösung entstanden ist und
c) diese Lösung abkühlt und auf einen pH-Wert von 4 bis 5 einstellt und sterilisiert.

## Claims

1. A process with whose aid basic active substances which have nitrogen atoms which can be protonated in acids can be processed together with phospholipids to give a homogeneous solution, which comprises
a) mixing the active substance with a phospholipid,
b) adding an acidic aqueous solution of a carbohydrate to the residue at elevated temperature, and stirring this mixture at elevated temperature until a homogeneous colloidal solution is produced, and
c) cooling this solution, and adjusting to a pH of 4-5 and sterilizing.

## Revendications

1. Procédé à l'aide duquel on peut transformer en une solution homogène, conjointement avec des phospholipides, des substances actives présentant des atomes d'azote basiques, protonables dans les acides, caractérisé par le fait que
a) on mélange la substance active avec un phospholipide,
b) on ajoute au résidu à température élevée une solution aqueuse acide d'un hydrate de carbone et on agite ce mélange à température élevée jusqu'à ce qu'il se forme une solution colloïdale homogène et
c) on refroidit cette solution et on ajuste à une valeur de pH de 4 à 5 et on stérilise.
